(19) Europäisches Patentamt
European Patent Office
Office européen des brevets

(11) EP 3 191 075 B1

(12) EUROPEAN PATENT SPECIFICATION

(45) Date of publication and mention of the grant of the patent:
**15.02.2023 Bulletin 2023/07**

(21) Application number: **15759455.7**

(22) Date of filing: **04.09.2015**

(51) International Patent Classification (IPC):
***A61K 8/37*** *(2006.01)* ***A61K 8/49*** *(2006.01)*
***A61Q 17/04*** *(2006.01)* ***A61P 17/16*** *(2000.01)*
***A61P 43/00*** *(2000.01)*

(52) Cooperative Patent Classification (CPC):
**A61K 8/496; A61K 8/37; A61K 8/4966; A61P 17/16; A61P 43/00; A61Q 17/04;**
A61K 2800/592

(86) International application number:
**PCT/EP2015/070257**

(87) International publication number:
**WO 2016/037942 (17.03.2016 Gazette 2016/11)**

(54) **MIXTURES OF COSMETIC UV ABSORBERS**

MISCHUNGEN AUS KOSMETISCHEN UV-ABSORBERN

MÉLANGES D’ABSORBEURS D’UV POUR COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2014 EP 14184028**

(43) Date of publication of application:
**19.07.2017 Bulletin 2017/29**

(73) Proprietor: **BASF SE**
**67056 Ludwigshafen (DE)**

(72) Inventors:
- **GRUMELARD, Julie**
  **F-68128 Village-Neuf (FR)**
- **EHLIS, Thomas**
  **79100 Freiburg (DE)**
- **FLOESSER-MUELLER, Heike**
  **B-2930 Brasschaat (BE)**

(74) Representative: **BASF IP Association**
**BASF SE**
**G-FLP-C006**
**67056 Ludwigshafen (DE)**

(56) References cited:
**EP-A1- 2 078 521** **WO-A1-2016/012586**
**US-A- 4 457 911** **US-A1- 2006 052 491**

- **DATABASE ChemSpider [Online] "benzylidene malonate", XP002739828, Database accession no. 813477**
- **HERZOG: INTERNATIONAL JOURNAL FOR APPLIED SCIENCE, vol. 133, no. 7-2007, 2007, pages 26-25,**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 191 075 B1

## Description

The present invention relates to the use of UV filter combinations comprising a specific benzotriazole derivative for cosmetic preparations.

It is well known that ultraviolet radiation (light) is harmful to human skin. Depending on the wavelength UV radiation causes different types of skin damage. UV-B radiation (about 290 to about 320 nm) is responsible for sunburn and can cause skin cancer. UV-A radiation (about 320 to about 400 nm) while producing tanning of the skin, contributes also to sunburn and the induction of skin cancers. Moreover, the harmful effects of the UV-B radiation may be aggravated by UV-A radiation.

US 2006/0052491 describes a stabilizer mixture comprising specific compounds of the triazine class and benzotriazoles for stabilizing organic materials against degradation induced by light, heat or oxidation.

EP 2078521 A1 describes cosmetic formulations comprising a combination of two UV filters of a benzotriazole derivative and an aryl hydrocarbon receptor antagonist.

US 4,457,911 describes a sunscreen formulation which contains a substituted dialkyl malonate as an adjuvant for a specific group of organic UV filters.

Surprisingly it has been found that a specific benzotriazole derivative has very good stabilizing properties in sunscreen compositions in combination with another specific UV filter.

Therefore, the present invention relates to the use of the benzotriazole derivative (a) corresponding to the formula

(1) 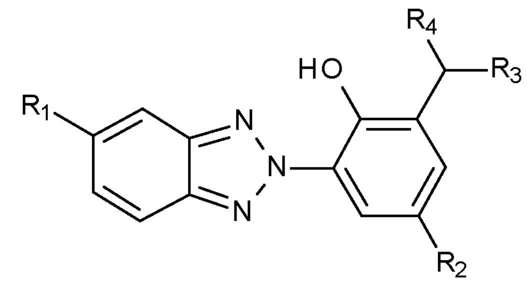

wherein

$R_1$ is hydrogen; $C_1$-$C_5$ alkyl; $C_1$-$C_5$ alkoxy; or halogen;
$R_2$ is hydrogen; $C_1$-$C_{20}$ alkyl; $C_1$-$C_5$ alkoxy; $C_1$-$C_5$ alkoxycarbonyl; $C_5$-$C_{10}$ cycloalkyl; $C_5$-$C_{10}$ aryl; or $C_5$-$C_{10}$ aralkyl;
$R_3$ is $C_1$-$C_{20}$ alkyl; $C_5$-$C_{10}$ cycloalkyl; $C_1$-$C_{20}$ alkoxy; or $C_5$-$C_{10}$ cycloalkoxy; and
$R_4$ is hydrogen; or $C_1$-$C_5$ alkyl;
for photo-stabilizing cosmetic formulations comprising at least one of the UV filters ($c_6$) Ethylhexyl Methoxycinnamate; and
($b_1$) benzylidene malonates UV.

The term "$C_x$-$C_y$alkyl" as used herein refers to straight-chain or branched alkyl radicals having x to y carbon atoms such as e.g. methyl, ethyl, n-propyl, 1-methylethyl, n-butyl, 1-methylpropyl, 2-methylpropyl, 1, 1-dimethylethyl, n-pentyl, 1-methylbutyl, 2-methylbutyl, 3-methylbutyl, 2,2-dimethylpropyl, 1-ethylpropyl, n-hexyl, 1, 1-dimethylpropyl, 1,2-dimethylpropyl, 1-methylpentyl, 2-methylpentyl, 3-methylpentyl, 4-methylpentyl, 1, 1-dimethylpropyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,2,2-trimethylpropyl, 1-ethyl-1-methylpropyl, 1-ethyl-2-methylpropyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl or n-eicosyl without being limited thereto.

The term $C_5$-$C_{10}$ cycloalkyl denotes to unsubstituted or $C_1$-$C_{10}$alkyl (mono- or poly-)substituted, in particular $C_1$-$C_5$ alkyl (mono- or poly-)substituted cyclic, bicyclic or tricyclic hydrocarbon residues such as in particular cyclopentyl, cyclohexyl, cycloheptyl or decahydronaphtyl. Preferably, $C_5$-$C_{10}$cycloalkyl denotes to unsubstituted or $C_1$-$C_2$alkyl (mono- or poly-)substituted cyclopentyl, cyclohexyl or cycloheptyl such as in particular to unsubstituted or (mono- or poly-)methyl substituted cyclohexyl such as most in particular cyclohexyl or 3,3,5-trimethylcyclohexyl.

The term "$C_x$-$C_y$ alkoxy" as used herein denotes to linear or branched alkoxy-, respectively unsubstituted or (mono- or poly-)substituted cycloalkoxy radicals having from x to y carbon atoms such as e.g. methoxy, ethoxy, propoxy, butyloxy or pentyloxy, 2,5,5-trimethylhexyloxy, 3,5,5-trimethylhexyloxy, isoamyloxy, 2-ethylhexyloxy or 3,3,5-trimethylcyclohexyloxy.

The term $C_6$-$C_{10}$ aryl refers e.g. to naphthyl or phenyl radicals, preferably phenyl.

A cosmetic or pharmaceutical composition containing the above UV absorber mixture is especially useful for

the protection of organic materials that are sensitive to ultraviolet light, especially human and animal skin and hair, against the action of UV radiation. Such UV filter combinations are therefore suitable as light-protective agents in cosmetic, pharmaceutical and veterinary medicine preparations.

The cosmetic or pharmaceutical composition contains from 0.1 to 15 % by weight, preferably from 0.5 to 10 % by weight, based on the total weight of the composition, of the UV absorber mixture of (a) and ($b_1$), (a) and ($c_6$) or (a), ($b_1$) and ($c_6$) and a cosmetically tolerable adjuvant.

The cosmetic or pharmaceutical composition can be prepared by physically mixing the UV absorbers with the adjuvant using customary methods, for example by simply stirring together the individual components, especially by making use of the dissolution properties of already known cosmetic UV absorbers, for example Ethylhexyl Methoxycinnamate. The UV absorbers can be used, for example, without further treatment.

In addition to other properties, the cosmetic or pharmaceutical composition can be used as a radical scavenger by reducing significantly the number of UV- induced free radicals in skin when applied in a suitable cosmetic carrier.

The cosmetic composition may comprise, in addition to the UV absorber combination used according to the present invention, one or more further UV protective agents of the following substance classes:

p-aminobenzoic acid derivatives, salicylic acid derivatives, benzophenone derivatives, 3-imidazol-4-yl acrylic acid and esters; benzofuran derivatives, polymeric UV absorbers, camphor derivatives, encapsulated UV absorbers, and 4,4-diphenyl-1,3-butadiene derivatives.

Special preference is given to the light-protective agents indicated in the following Table 3:

Table 3: Suitable UV filter substances and adjuvants which can be additionally used with the UV absorbers used according to the present invention

| Chemical Name | CAS No. |
|---|---|
| (+/-)-1,7,7-trimethyl-3-[(4-methylphenyl)methylene]bicyclo[2.2.1]heptan-2-one; p-methyl benzylidene camphor | 36861-47-9 |
| 1,7,7-trimethyl-3-(phenylmethylene)bicyclo[2.2.1]heptan-2-one; benzylidene camphor | 15087-24-8 |
| (2-Hydroxy-4-methoxyphenyl)(4-methylphenyl)methanone | 1641-17-4 |
| 2,4-dihydroxybenzophenone | 131-56-6 |
| 2,2',4,4'-tetrahydroxybenzophenone | 131-55-5 |
| 2-Hydroxy-4-methoxy benzophenone; | 131-57-7 |
| 2,2'-dihydroxy-4,4'-dimethoxybenzophenone | 131-54-4 |
| 2,2'-Dihydroxy-4-methoxybenzophenone | 131-53-3 |
| Alpha-(2-oxoborn-3-ylidene)toluene-4-sulphonic acid and its salts (Mexoryl SL) | 56039-58-8 |
| Methyl N,N,N-trimethyl-4-[(4,7,7-trimethyl-3-oxobicyclo[2,2,1]hept-2-ylidene)methyl] anilinium sulphate (Mexoryl SO) | 52793-97-2 |
| Isopentyl p-Methoxycinnamate; isoamyl Methoxycinnamate | 71617-10-2 |
| Menthyl-o-aminobenzoate | 134-09-8 |
| Menthyl salicylate | 89-46-3 |
| 4- aminobenzoic acid | 150-13-0 |
| Benzoic acid, 4-amino-, ethyl ester, polymer with oxirane | 113010-52-9 |
| 2-Propenamide, N-[[4-[(4,7,7-trimethyl-3-oxobicyclo[2.2.1]hept-2-ylidene)methyl] phenyl]methyl]-, homopolymer | 147897-12-9 |
| Triethanolamine salicylate | 2174-16-5 |
| 3, 3'-(1,4-phenylenedimethylene)bis[7, 7-dimethyl- 2-oxo-bicyclo[2.2.1]heptane-1 methanesulfonic acid] (Cibafast H) | 90457-82-2 |
| Zinc oxide (primary particle size 20-100 nm) For example Zinc oxide NDM, Zinc oxide Z-Cote HP1, Nanox Zinc oxide | 1314-13-2 |

(continued)

| Table 3: Suitable UV filter substances and adjuvants which can be additionally used with the UV absorbers used according to the present invention | |
|---|---|
| Chemical Name | CAS No. |
| Benzoic acid, 4,4'-[[6-[[4-[[(1,1-dimethylethyl)amino]carbonyl]-phenyl]amino]1,3,5-triazine-2,4-diyl]diimino]bis-, bis(2-ethylhexyl)ester; diethylhexyl butamido triazone (Uvasorb HEB) | 154702-15-5 |
| Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| Dimethicodiethylbenzalmalonate; Polysilicone 15 (Parsol SLX) | 207574-74-1 |
| Benzenesulfonic acid, 3-(2H-benzotriazol-2-yl)-4-hydroxy-5-(1-methylpropyl)-, monosodium salt (Tinogard HS) | 92484-48-5 |
| 1-Dodecanaminium, N-[3-[[4-(dimethylamino)benzoyl]amino]propyl]N,N-dimethyl-, salt with 4-methylbenzenesulfonic acid (1:1) (Escalol HP610) | 156679-41-3 |
| 1-Propanaminium, N,N,N-trimethyl-3-[(1-oxo-3-phenyl-2-propenyl)-amino]-, chloride | 177190-98-6 |
| 1H-Benzimidazole-4,6-disulfonic acid, 2,2'-(1,4-phenylene)bis- | 170864-82-1 |
| 1,3,5-Triazine, 2,4,6-tris(4-methoxyphenyl)- | 7753-12-0 |
| 1,3,5-Triazine, 2,4,6-tris[4-[(2-ethylhexyl)oxy]phenyl]- | 208114-14-1 |
| 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 2-Propenoic acid, 3-(1H-imidazol-4-yl)- | 104-98-3 |
| Benzoic acid, 2-hydroxy-, [4-(1-methylethyl)phenyl]methyl ester | 94134-93-7 |
| 1,2,3-Propanetriol, 1-(4-aminobenzoate) (Glyceryl PABA) | 136-44-7 |
| Benzeneacetic acid, 3,4-dimethoxy-a-oxo- | 4732-70-1 |
| 2-Propenoic acid, 2-cyano-3,3-diphenyl-, ethyl ester | 5232-99-5 |
| Anthralinic acid, p-menth-3-yl ester | 134-09-8 |
| 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7 |
| sterols (cholesterol, lanosterol, phytosterols), as described in WO0341675 | |
| mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes) | |
| alpha-lipoic-acid as described in DE 10229995 | |
| synthetic organic polymers as described in EP 1 371 358, [0033]-[0041] | |
| phyllosilicates as described in EP 1371357 [0034]-[0037] | |
| silica compounds as described in EP1371356, [0033]-[0041] | |
| inorganic particles as described in DE10138496 [0043]-[0055] | |
| latex particles as described in DE10138496 [0027]-[0040] | |
| 1H-Benzimidazole-4,6-disulfonic acid, 2,2'-(1,4-phenylene)bis-, disodium salt; Bisimidazylate (Neo Heliopan APC) | 180898-37-7 |
| Di-2-ethylhexyl-3,5-dimethoxy-4-hydroxy-benzalmalonate (Oxynex ST, EMD Chemicals, as described in US 20040247536) | |
| Z-COTE® MAX: Zinc Oxide (and) Diphenyl Capryl Methicone | |

(continued)

| Table 3: Suitable UV filter substances and adjuvants which can be additionally used with the UV absorbers used according to the present invention | |
|---|---|
| Chemical Name | CAS No. |
| Z-COTE HP1: Zinc Oxide (and) Triethoxycaprylylsilane | |
| 1,3,5-Triazine-2,4,6-triamine, N2,N4-bis[4-[5-(1,1-dimethylpropyl)-2-benzoxazolyl] phenyl]-N6-(2-ethylhexyl)- (Uvasorb K2A) | 288254-16-0 |
| 1,1-[(2,2'-Dimethylpropoxy)carbonyl]-4,4-diphenyl-1,3-butadiene | 363602-15-7 |
| UV filter capsules containing an organic sunscreen as described in DE102007035567 or WO 2009012871 | |

If the compositions represent water- and oil-containing emulsions (e.g. W/O, O/W, O/W/O and W/O/W emulsions or microemulsions) they contain, for example, from 0.1 to 30 % by weight, preferably from 0.1 to 15 % by weight and especially from 0.5 to 10 % by weight, based on the total weight of the composition, of the mixture of components of (a) and ($b_1$), (a) and ($c_6$) or (a), ($b_1$) and ($c_6$) from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight and preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically tolerable adjuvants.

Suitable oil components of oil-containing compositions (e.g. oils, W/O, O/W, O/W/O and W/O/W emulsions or microemulsions) are for example Guerbet alcohols based on fatty alcohols having from 6 to 18, preferably from 8 to 10, carbon atoms, esters of linear $C_6$-$C_{24}$ fatty acids with linear $C_3$-$C_{24}$ alcohols, esters of branched $C_6$-$C_{13}$carboxylic acids with linear $C_6$-$C_{24}$ fatty alcohols, esters of linear $C_6$-$C_{24}$ fatty acids with branched alcohols, especially 2-ethylhexanol, esters of hydroxycarboxylic acids with linear or branched $C_6$-$C_{22}$ fatty alcohols, especially dioctyl malates, esters of linear and/or branched fatty acids with polyhydric alcohols (for example propylene glycol, dimer diol or trimer triol) and/or Guerbet alcohols, triglycerides based on $C_6$-$C_{10}$ fatty acids, liquid mono-/di-/tri-glyceride mixtures based on $C_6$-$C_{18}$ fatty acids, esters of $C_6$-$C_{24}$ fatty alcohols and/or Guerbet alcohols with aromatic carboxylic acids, especially benzoic acid, esters of $C_2$-$C_{12}$dicarboxylic acids with linear or branched alcohols having from 1 to 22 carbon atoms or polyols having from 2 to 10 carbon atoms and from 2 to 6 hydroxy groups, vegetable oils (such as sunflower oil, olive oil, soybean oil, rapeseed oil, almond oil, jojoba oil, orange oil, wheat germ oil, peach kernel oil and the liquid components of coconut oil), branched primary alcohols, substituted cyclohexanes, linear and branched $C_6$-$C_{22}$ fatty alcohol carbonates, Guerbet carbonates, esters of benzoic acid with linear and/or branched $C_6$-$C_{22}$alcohols (e.g. Finsolv® TN), linear or branched, symmetric or asymmetric dialkyl ethers having a total of from 12 to 36 carbon atoms, especially from 12 to 24 carbon atoms, for example di-n-octyl ether, di-n-decyl ether, di-n-nonyl ether, di-n-undecyl ether, di-n-dodecyl ether, n-hexyl n-octyl ether, n-octyl n-decyl ether, n-decyl n-undecyl ether, n-undecyl n-dodecyl ether, n-hexyl n-undecyl ether, di-tert-butyl ether, diisopentyl ether, di-3-ethyldecyl ether, tert-butyl n-octyl ether, isopentyl n-octyl ether and 2-methyl pentyl-n-octyl ether; ring-opening products of epoxidized fatty acid esters with polyols, silicone oils and/or aliphatic or naphthenic hydrocarbons. Also of importance are monoesters of fatty acids with alcohols having from 3 to 24 carbon atoms. That group of substances comprises the esterification products of fatty acids having from 8 to 24 carbon atoms, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petro-selinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols). Of special importance are isopropyl myristate, isono-nanoic acid $C_{16}$-$C_{18}$alkyl esters, stearic acid 2-ethylhexyl ester, cetyl oleate, glycerol tricaprylate, coconut fatty alcohol caprinate/caprylate and n-butyl stearate. Further oil components that can be used are dicarboxylic acid esters, such as di-n-butyl adipate, di(2-ethylhexyl) adipate, di(2-ethylhexyl) succinate and diisotridecyl acetate, and also diol esters, such as ethylene glycol

dioleate, ethylene glycol diisotridecanoate, propylene glycol di(2-ethylhexanoate), propylene glycol diisostearate, propylene glycol dipelargonate, butanediol diisostearate and neopentyl glycol dicapry-late. Preferred mono- or polyols are ethanol, isopropanol, propylene glycol, hexylene glycol, glycerol and sorbitol. It is also possible to use di- and/or trivalent metal salts (alkaline earth metal, $Al^{3+}$ *inter alia*) of one or more alkyl carboxylic acids.

The oil components can be used in an amount of, for example, from 1 to 60 % by weight, especially from 5 to 50 % by weight and preferably from 10 to 35 % by weight, based on the total weight of the composition.

Any conventionally usable emulsifier can be used for the cosmetic compositions.

Suitable emulsifiers are for example, non-ionic surfactants from the following groups:

- addition products of from 2 to 30 mol of ethylene oxide and/or from 0 to 5 mol of propylene oxide with linear fatty alcohols having from 8 to 22 carbon atoms, with fatty acids having from 12 to 22 carbon atoms and with alkylphenols having from 8 to 15 carbon atoms in the alkyl group, for example ceteareth-20 or ceteareth-12;
- $C_{12}$-$C_{22}$ fatty acid mono- and di-esters of addition products of from 1 to 30 mol of ethylene oxide with polyols having from 3 to 6 carbon atoms, especially with glycerol;
- glycerol mono- and di-esters and sorbitan mono- and di-esters of saturated and unsaturated fatty acids having from 6 to 22 carbon atoms and ethylene oxide addition products thereof, for example glyceryl stearates, glyceryl isostearates, glyceryl oleates, sorbitan oleates or sorbitan sesquioleates;
- $C_8$-$C_{22}$alkyl-mono- and -oligo-glycosides and ethoxylated analogues thereof, degrees of oligomerisation of from 1.1 to 5, especially from 1.2 to 1.4, being preferred, and glucose being preferred as the sugar component;
- addition products of from 2 to 60 mol, especially from 15 to 60 mol, of ethylene oxide with castor oil and/or hydrogenated castor oil;
- polyol esters and especially polyglycerol esters, for example diisostearoyl polyglyceryl-3-diisostearates, polyglyceryl-3-diisostearates, triglyceryl diisostearates, polyglyceryl-2-sesquiisostearates or polyglyceryl dimerates. Mixtures of compounds from a plurality of those substance classes are also suitable;
- partial esters based on linear, branched, unsaturated or saturated $C_6$-$C_{22}$ fatty acids, ricinoleic acid and also 12-hydroxystearic acid and on glycerol, polyglycerol, pentaerythritol, dipentaerythritol, sugar alcohols (e.g. sorbitol), alkyl glucosides (e.g. methyl glucoside, butyl glucoside, lauryl glucoside) and also polyglucosides (e.g. cellulose), for example polyglyceryl-2-dihydroxystearates or polyglyceryl-2-diricinoleates;
- mono-, di- and tri-alkyl phosphates and also mono-, di- and/or tri-PEG-alkyl phosphates and salts thereof;
- wool wax alcohols;
- one or more ethoxylated esters of natural derivatives, for example polyethoxylated esters of hydrogenated castor oil;
- silicone oil emulsifiers, for example silicone polyol;
- polysiloxane/polyalkyl/polyether copolymers and corresponding derivatives, for example cetyl dimethicone copolyol;
- mixed esters of pentaerythritol, fatty acids, citric acid and fatty alcohol (see DE-A-1 165574) and/or mixed esters of fatty acids having from 6 to 22 carbon atoms, methylglucose and polyols, preferably glycerol or polyglycerol, for example polyglyceryl-3-glucose distearates, polyglyceryl-3-glucose dioleates, methyl glucose dioleates or dicocoyl pentaerythryl distearyl citrates; and also
- polyalkylene glycols.

The addition products of ethylene oxide and/or of propylene oxide with fatty alcohols, fatty acids, alkylphenols, glycerol mono- and di-esters and also sorbitan mono- and di-esters of fatty acids, or with castor oil, are known, commercially available products. They are usually homologue mixtures, the average degree of alkoxylation of which corresponds to the ratio of the amounts of ethylene oxide and/or propylene oxide and substrate with which the addition reaction is carried out. $C_{12}$-$C_{18}$ fatty acid mono- and di-esters of addition products of ethylene oxide with glycerol are known, for example, from DE-A-2 024 051 as fat-restoring substances for cosmetic preparations.

$C_8$-$C_{18}$Alkyl-mono- and -oligo-glycosides, their preparation and their use are known from the prior art. They are prepared especially by reacting glucose or oligosaccharides with primary alcohols having from 8 to 18 carbon atoms. Suitable glycoside radicals include monoglycosides in which a cyclic sugar radical is glycosidically bonded to the fatty alcohol and also oligomeric glycosides having a degree of oligomerisation of up to preferably about 8. The degree of oligomerisation is a statistical average value based on a homologue distribution customary for such technical-grade products.

It is also possible to use zwitterionic surfactants as emulsifiers. The term "zwitterionic surfactants" denotes especially surface-active compounds that carry at least one quaternary ammonium group and at least one carboxylate and/or sulfonate group in the molecule. Zwitterionic surfactants that are especially suitable are the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines each having from 8 to 18 carbon atoms in the alkyl or acyl group and also cocoacylaminoethylhydroxyethylcarboxymethylglycinate. Special preference is given to the fatty acid amide derivative

known by the CTFA name cocamidopropyl betaine. Likewise suitable as emulsifiers are ampholytic surfactants. Ampholytic surfactants are to be understood as meaning especially those which, in addition to containing a $C_8$-$C_{18}$-alkyl or -acyl group, contain at least one free amino group and at least one -COOH or -$SO_3H$ group in the molecule and are capable of forming internal salts. Examples of suitable ampholytic surfactants include N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids, each having approximately from 8 to 18 carbon atoms in the alkyl group.

Ampholytic surfactants to which special preference is given are N-cocoalkylamino-propionate, cocoacylaminoethylaminopropionate and $C_{12}$-$C_{18}$acylsarcosine. In addition to the ampholytic emulsifiers there also come into consideration quaternary emulsifiers, special preference is given to those of the esterquat type, preferably methyl-quaternised di-fatty acid triethanolamine ester salts.

Non-ionic emulsifiers are preferred, preferably ethoxylated fatty alcohols having from 8 to 22 carbon atoms and from 4 to 30 EO units.

The emulsifiers may be used in an amount of, for example, from 1 to 30 % by weight, especially from 4 to 20 % by weight and preferably from 5 to 10 % by weight, based on the total weight of the composition. It is, however, also possible in principle to dispense with the use of emulsifiers.

The compositions, for example creams, gels, lotions, alcoholic and aqueous/alcoholic solutions, emulsions, wax/fat compositions, stick preparations, powders or ointments, may in addition contain, as further adjuvants and additives, mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, antioxidants, hydrotropic agents, preservatives, insect repellents, self-tanning agents, solubilizers, perfume oils, colorants, bacteria-inhibiting agents and the like.

Substances suitable for use as super-fatting agents are, for example, lanolin and lecithin and also polyethoxylated or acrylated lanolin and lecithin derivatives, polyol fatty acid esters, monoglycerides and fatty acid alkanolamides, the latter simultaneously acting as foam stabilisers.

Examples of suitable mild surfactants, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, $\alpha$-olefin sulfonates, ether carboxylic acids, alkyl oligoglucosides, fatty acid glucamides, alkylamidobetaines and/or protein fatty acid condensation products, the latter preferably being based on wheat proteins.

Suitable pearlescent are for example: alkylene glycol esters, especially ethylene glycol distearate; fatty acid alkanolamides, especially coco fatty acid diethanolamide; partial glycerides, especially stearic acid monoglyceride; esters of polyvalent, unsubstituted or hydroxy-substituted carboxylic acids with fatty alcohols having from 6 to 22 carbon atoms, especially long-chained esters of tartaric acid; fatty substances, for example fatty alcohols, fatty ketones, fatty aldehydes, fatty ethers and fatty carbonates, which in total have at least 24 carbon atoms, especially laurone and distearyl ether; fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures thereof.

Suitable consistency regulators are especially fatty alcohols or hydroxy fatty alcohols having from 12 to 22 carbon atoms and preferably from 16 to 18 carbon atoms, and in addition partial glycerides, fatty acids and hydroxy fatty acids. Preference is given to a combination of such substances with alkyl-oligoglucosides and/or fatty acid N-methylglucamides of identical chain length and/or polyglycerol poly-12-hydroxystearates. Suitable thickeners include, for example, Aerosil types (hydrophilic silicic acids), polysaccharides, especially xanthan gum, guar-guar, agar-agar, alginates and Tyloses, carboxymethyl cellulose and hydroxymethyl cellulose, also relatively high molecular weight polyethylene glycol mono- and di-esters of fatty acids, polyacrylates (e.g. Carbopol® from Goodrich or Synthalen® from Sigma), polyacrylamides, polyvinyl alcohol and polyvinylpyrrolidone, surfactants, for example ethoxylated fatty acid glycerides, esters of fatty acids with polyols, for example pentaerythritol or trimethylolpropane, fatty alcohol ethoxylates with restricted homologue distribution and alkyl-oligoglucosides as well as electrolytes, such as sodium chloride or ammonium chloride.

Suitable cationic polymers are, for example, cationic cellulose derivatives, for example a quarter-nised hydroxymethyl cellulose obtainable under the name Polymer JR 400® from Amerchol, cationic starch, copolymers of diallylammonium salts and acrylamides, quaternised vinylpyrrolidone/- vinyl imidazole polymers, for example Luviquat® (BASF), condensation products of polyglycols and amines, quaternised collagen polypeptides, for example lauryldimonium hydroxypropyl hydrolyzed collagen (Lamequat®L/Grünau), quaternised wheat polypeptides, polyethyleneimine, cationic silicone polymers, for example amidomethicones, copolymers of adipic acid and di-methylaminohydroxypropyldiethylenetriamine (Cartaretin®/Sandoz), copolymers of acrylic acid with dimethyldiallylammonium chloride (Merquat® 550/Chemviron), polyaminopolyamides, as described, for example, in FR-A-2 252 840, and the crosslinked water-soluble polymers thereof, cationic chitin derivatives, for example quaternised chitosan, optionally distributed as microcrystals; condensation products of dihaloalkyls, for example dibromobutane, with bisdialkyl-amines, for example bisdimethylamino-1,3-propane, cationic guar gum, for example Jaguar® C-17, Jaguar® C-16 from Celanese, quaternised ammonium salt polymers, for example Mirapol® A-15, Mirapol® AD-1, Mirapol® AZ-1 from Miranol.

Suitable anionic, zwitterionic, amphoteric and non-ionic polymers are for example, vinyl acetate/- crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids and polyacrylic acids crosslinked with polyols, acrylami-dopropyltrimethylammonium chloride/acrylate copolymers, octyl acrylamide/methyl methacrylate/tert-butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinyl caprolactam terpolymers and also optionally derivatised cellulose ethers and silicones.

Suitable silicone compounds are, for example, dimethylpolysiloxanes, methylphenylpolysiloxanes, cyclic silicones, and also amino-, fatty acid-, alcohol-, polyether-, epoxy-, fluorine-, glycoside- and/or alkyl-modified silicone compounds, which at room temperature may be in either liquid or resinous form. Also suitable are simethicones, which are mixtures of dimethicones having an average chain length of from 200 to 300 dimethylsiloxane units with hydrogenated silicates. A detailed survey by Todd et al. of suitable volatile silicones may in addition be found in Cosm. Toil. 91, 27 (1976).

Typical examples of fats are glycerides, and as waxes there come into consideration, *inter alia,* beeswax, carnauba wax, candelilla wax, montan wax, paraffin wax, hydrogenated castor oils and fatty acid esters or microwaxes solid at room temperature optionally in combination with hydrophilic waxes, e.g. cetylstearyl alcohol or partial glycerides. Metal salts of fatty acids, for example magnesium, aluminium and/or zinc stearate or ricinoleate, may be used as stabilizers.

Biogenic active ingredients are to be understood as meaning, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Suitable deodorizing active ingredients are for example, antiperspirants like aluminium chlorohydrates (see J. Soc. Cosm. Chem. 24, 281 (1973)). Aluminium chlorohydrate corresponding to formula $Al_2(OH)_5Cl$ x 2.5 $H_2O$, known and commercially available under the trade mark Locron® of Hoechst AG, Frankfurt (FRG), is especially preferred (see J. Pharm. Pharmacol. 26, 531 (1975)). Beside the chlorohydrates, it is also possible to use aluminium hydroxy-acetates and acidic aluminium/zirconium salts. Esterase inhibitors may be added as further deodorising active ingredients. Such inhibitors are preferably trialkyl citrates, such as trimethyl citrate, tripropyl citrate, triisopropyl citrate, tributyl citrate and especially triethyl citrate (Hydagen® CAT, Henkel KGaA, Düsseldorf/FRG), which inhibit enzyme activity and hence reduce odour formation. Further suitable esterase inhibitors are sterol sulfates or phosphates, for example lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester and hydroxycarboxylic acids and esters thereof, for example citric acid, malic acid, tartaric acid or tartaric acid diethyl ester. Antibacterial active ingredients that influence the microbial flora and kill, or inhibit the growth of, sweat-decomposing bacteria can likewise be present in the preparations (especially in stick preparations). Examples include chitosan, phenoxyethanol and chlorhexidine gluconate. 5-Chloro-2-(2,4-dichlorophenoxy)-phenol (Irgasan®, BASF has also proved especially effective.

Suitable anti-dandruff agents are for example, climbazole, octopirox and zinc pyrithione. Customary film formers include, for example, chitosan, microcrystalline chitosan, quaternised chitosan, polyvinylpyrrolidone, vinylpyrrolidone/vinyl acetate copolymers, polymers of quaternary cellulose derivatives containing a high proportion of acrylic acid, collagen, hyaluronic acid and salts thereof and similar compounds. Suitable swelling agents for aqueous phases are montmorillonites, clay mineral substances, Pemulen and also alkyl-modified types of Carbopol (Goodrich). Further suitable polymers and swelling agents can be found in the review by R. Lochhead in Cosm. Toil. 108, 95 (1993).

In addition to the primary light-protective substances it is also possible to use secondary light-protective substances of the antioxidant type which interrupt the photochemical reaction chain triggered when UV radiation penetrates the skin or hair. Typical examples of such antioxidants are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. $\alpha$-carotene, $\beta$-carotene, lycopene) and derivatives thereof, chlorogenic acid and derivatives thereof, lipoic acid and derivatives thereof (e.g. dihydrolipoic acid), aurothioglycose, propylthiouracil and other thiols (e.g. thioredoxin, glutathione, cysteine, cystine, cystamine and the glycosyl, N-acetyl, methyl, ethyl, propyl, amyl, butyl, lauryl, palmitoyl, oleyl, $\gamma$-linoleyl, cholesteryl and glyceryl esters thereof) and also salts thereof, dilauryl thiodipropionate, distearyl thiodipropionate, thiodipropionic acid and derivatives thereof (esters, ethers, peptides, lipids, nucleotides, nucleosides and salts) and also sulfoximine compounds (e.g. buthionine sulfoximines, homocysteine sulfoximine, buthionine sulfones, penta-, hexa-, hepta-thionine sulfoximine) in very small tolerable amounts (e.g. from pmol to $\mu$mol/kg), also (metal) chelating agents (e.g. $\alpha$-hydroxy fatty acids, palmitic acid, phytic acid, lactoferrin), $\alpha$-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. $\gamma$-linolenic acid, linoleic acid, oleic acid), folic acid and derivatives thereof, ubiquinone and ubiquinol and derivatives thereof, vitamin

C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate) and also coniferyl benzoate of benzoin resin, rutinic acid and derivatives thereof, α-glycosylrutin, ferulic acid, furfurylidene glucitol, carnosine, butyl hydroxytoluene, butyl hydroxyanisole, resinous nordihydroguaiaretic acid, nordihydroguaiaretic acid, trihydroxybutyrophenone, uric acid and derivatives thereof, mannose and derivatives thereof, superoxide dismutase, N-[3-(3,5-di-tert-butyl-4-hydroxyphenyl)propionyl]sulfanilic acid (and salts thereof, for example the sodium salts), zinc and derivatives thereof (e.g. ZnO, $ZnSO_4$), selenium and derivatives thereof (e.g. selenium methionine), stilbene and derivatives thereof (e.g. stilbene oxide, trans-stilbene oxide) and the derivatives thereof (salts, esters, ethers, sugars, nucleotides, nucleosides, peptides and lipids) of those mentioned active ingredients. HALS (="Hindered Amine Light Stabilizers") compounds may also be mentioned. The amount of antioxidants present is usually from 0.001 to 30 % by weight, preferably from 0.01 to 3 % by weight, based on the weight of the cosmetic composition.

For improvement of the flow behavior it is also possible to employ hydrotropic agents, for example ethanol, isopropyl alcohol or polyols. Suitable polyols for that purpose comprise preferably from 2 to 15 carbon atoms and at least two hydroxy groups.

The polyols may also contain further functional groups, especially amino groups, and/or may be modified with nitrogen. Typical examples are as follows:

- glycerol;
- alkylene glycols, for example ethylene glycol, diethylene glycol, propylene glycol, butylene glycol, hexylene glycol and also polyethylene glycols having an average molecular weight of from 100 to 1000 Dalton;
- technical oligoglycerol mixtures having an intrinsic degree of condensation of from 1.5 to 10, for example technical diglycerol mixtures having a diglycerol content of from 40 to 50 % by weight;
- methylol compounds, such as, especially, trimethylolethane, trimethylolpropane, trimethylolbutane, pentaerythritol and dipentaerythritol;
- lower alkyl-glucosides, especially those having from 1 to 8 carbon atoms in the alkyl radical, for example methyl and butyl glucoside;
- sugar alcohols having from 5 to 12 carbon atoms, for example sorbitol or mannitol;
- sugars having from 5 to 12 carbon atoms, for example glucose or saccharose;
- amino sugars, for example glucamine;
- dialcohol amines, such as diethanolamine or 2-amino-1,3-propanediol.

Suitable preservatives include, for example, phenoxyethanol, formaldehyde solution, Parabens, pentandiol or sorbic acid and the further substance classes listed in Schedule 6, Parts A and B of the Cosmetics Regulations.

Suitable perfume oils are mixtures of natural and/or synthetic aromatic substances. Representatives of natural aromatic substances are, for example, extracts from blossom (lilies, lavender, roses, jasmine, neroli, ylang-ylang), from stems and leaves (geranium, patchouli, petitgrain), from fruit (aniseed, coriander, carraway, juniper), from fruit peel (bergamot, lemons, oranges), from roots (mace, angelica, celery, cardamom, costus, iris, calmus), from wood (pinewood, sandalwood, guaiacum wood, cedarwood, rosewood), from herbs and grasses (tarragon, lemon grass, sage, thyme), from needles and twigs (spruce, pine, Scots pine, mountain pine), from resins and balsams (galbanum, elemi, benzoin, myrrh, olibanum, opoponax). Animal raw materials also come into consideration, for example civet and castoreum. Typical synthetic aromatic substances are, for example, products of the ester, ethers, aldehydes, ketones, alcohols or hydrocarbon types.

Aromatic substance compounds of the ester type are, for example, benzyl acetate, phenoxy-ethyl isobutyrate, p-tert-butylcyclohexyl acetate, linalyl acetate, dimethyl-benzylcarbinyl acetate, phenylethyl acetate, linalyl benzoate, benzyl formate, ethylmethylphenyl glycinate, allylcyclohexyl propionate, styrallyl propionate and benzyl salicylate. The ethers include, for example, benzyl ethyl ether; the aldehydes include, for example, the linear alkanals having from 8 to 18 hydrocarbon atoms, citral, citronellal, citronellyl oxyacetaldehyde, cyclamen aldehyde, hydroxycitro-nellal, lilial and bourgeonal; the ketones include, for example, the ionones, α-isomethylionone and methyl cedryl ketone; the alcohols include, for example, anethol, citronellol, eugenol, isoeugenol, geraniol, linalool, phenyl ethyl alcohol and terpinol; and the hydrocarbons include mainly the terpenes and balsams. It is preferable, however, to use mixtures of various aromatic substances that together produce an attractive scent. Ethereal oils of relatively low volatility, which are chiefly used as aroma components, are also suitable as perfume oils, e.g. sage oil, camomile oil, clove oil, melissa oil, oil of cinnamon leaves, lime blossom oil, juniper berry oil, vetiver oil, olibanum oil, galbanum oil, labolanum oil and lavandin oil. Preference is given to the use of bergamot oil, di-hydromyrcenol, lilial, lyral, citronellol, phenyl ethyl alcohol, α-hexyl cinnamaldehyde, geraniol, benzyl acetone, cyclamen aldehyde, linalool, boisambrene forte, ambroxan, indole, hedione, sandelice, lemon oil, tangerine oil, orange oil, allyl amyl glycolate, cyclovertal, lavandin oil, muscatel sage oil, β-damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat alone or in admixture with one another.

As colourants the substances that are suitable and permitted for cosmetic purposes, as compiled, for example, in the publication "Kosmetische Färbemittel" of the Farbstoffkommission der Deutschen Forschungsgemeinschaft, Verlag Chemie, Weinheim, 1984, pages 81 to 106 may be used. The colourants are usually used in concentrations of from 0.001 to 0.1 % by weight, based on the total mixture.

Typical examples of bacteria-inhibiting agents are preservatives that have a specific action against gram-positive bacteria, such as 2,4,4'-trichloro-2'-hydroxydiphenyl ether, chlorhexidine (1,6-di(4-chlorophenyl-biguanido)hexane) or TCC (3,4,4'-trichlorocarbanilide).

A large number of aromatic substances and ethereal oils also have antimicrobial properties. Typical examples are the active ingredients eugenol, menthol and thymol in clove oil, mint oil and thyme oil. A natural deodorizing agent of interest is the terpene alcohol farnesol (3,7,11-trimethyl-2,6,10-dodecatrien-1-ol), which is present in lime blossom oil. Glycerol monolaurate has also proved to be a bacteriostatic agent. The amount of the additional bacteria-inhibiting agents present is usually from 0.1 to 2 % by weight, based on the solids content of the cosmetic composition.

The cosmetic compositions may furthermore contain as adjuvants anti-foams, such as silicones, structurants, such as maleic acid, solubilizers, such as ethylene glycol, propylene glycol, glycerol or diethylene glycol, opacifiers, such as latex, styrene/PVP or styrene/acrylamide copolymers, complexing agents, such as EDTA, NTA, β-alaninediacetic acid or phosphonic acids, propellants, such as propane/butane mixtures, $N_2O$, dimethyl ether, $CO_2$, $N_2$ or air, so-called coupler and developer components as oxidation dye precursors, thioglycolic acid and derivatives thereof, thiolactic acid, cysteamine, thiomalic acid or α-mercaptoethanesulfonic acid as reducing agents or hydrogen peroxide, potassium bromate or sodium bromate as oxidizing agents.

Insect repellents are for example, N,N-diethyl-m-toluamide, 1,2-pentanediol or insect repellent 3535.

Suitable self-tanning agents are dihydroxyacetone, erythrulose or mixtures of dihydroxyacetone and erythrulose.

The UV filter combinations used according to the invention are contained in cosmetic formulations which form a wide variety of cosmetic preparations, especially the following preparations:

- skin-care preparations, e.g. skin-washing and cleansing preparations in the form of tablet-form or liquid soaps, synthetic detergents or washing pastes,
- bath preparations, e.g. liquid (foam baths, milks, shower preparations) or solid bath preparations, e.g. bath cubes and bathsalts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eye shadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, parfume), parfume oils or parfume creams;
- cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile.

The final formulations may exist in a wide variety of presentation forms, for example:

- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellant gas or pump-action spray) or an aerosol,
- in the form of a foam, or
- in the form of a paste.

Important cosmetic compositions for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection oils, sun protection milks and sun protection preparations in the form of a spray.

Important cosmetic compositions for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition:

0.01 to 5 % by weight of a UV absorber composition used according to the invention,
12.0 % by weight of sodium laureth-2-sulfate,
4.0 % by weight of cocamidopropyl betaine,
3.0 % by weight of sodium chloride, and
water ad 100 %.

Especially the following hair-cosmetic formulations may be used:

$a_1$) spontaneously emulsifying stock formulation, consisting of the UV absorbers used according to the invention, PEG-6-$C_{10}$oxoalcohol and sorbitan sesquioleate, to which water and any desired quaternary ammonium compound, for example 4 % minkamidopropyl-dimethyl-2-hydroxyethylammonium chloride or Quaternium 80 is added;
$a_2$) spontaneously emulsifying stock formulation consisting of the UV absorbers used according to the invention, tributyl citrate and PEG-20-sorbitan monooleate, to which water and any desired quaternary ammonium compound, for example 4 % minkamidopropyl-dimethyl-2-hydroxyethylammonium chloride or Quaternium 80 is added;
b) Quat-doped solutions of the UV absorbers used according to the invention in butyl triglycol and tributyl citrate;
c) mixtures or solutions of the UV absorbers used according to the invention with n-alkylpyrrolidone.

The specific UV filter combinations used according to the invention of (a) and ($b_1$), (a) and ($c_6$) or (a), ($b_1$) and ($c_6$) represent photo-stable cosmetic or pharmaceutical composition.

Thus, the present invention relates to the use of

(a) 2-(2H-Benzotriazol-2-yl)-6-(2-ethylhexyloxymethyl)-4-methyl-phenol for photo-stabilizing cosmetic formulations comprising at least one of the UV filters ($c_6$) Ethylhexyl Methoxycinnamate; and
($b_1$) the compound of formula

MBM-09

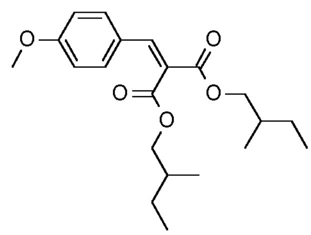

A. PREPARATION EXAMPLES

| | INCI Name | Composition | |
|---|---|---|---|
| | | Ex-01 | Ex-02 |
| Part A | Potassium Cetyl Phosphate | 1.80 | 1.80 |
| | Glyceryl Stearate | 2.5 | 2.5 |
| | Stearyl Alcohol | 2.5 | 2.5 |
| | C12-15 Alkyl Benzoate | 15 | 15 |
| | 2-(2H-Benzotriazol-2-yl)-6-(2-ethylhexyloxy-methyl)-4-methyl-phenol (= BTD-01) | 2.0 | 2.0 |
| | Ethylhexyl Methoxycinnamate | 10 | |
| | Compound MBM-09 *) | | 10 |
| Part B | Aqua | Qsp to 100 | Qsp to 100 |
| | | 60.61 | 60.61 |
| | | | |
| | Glycerin | 10.0 | 10.0 |
| | Xanthan Gum | 0.3 | 0.3 |
| Part C | Phenoxyethanol (and) Methylparaben (and) Ethylparaben (and)Butylparaben (and) Propylparaben (and) Isobutylparaben | 0.8 | 0.8 |
| pH before adjustment | | 5.6 | 5.54 |
| *) MBM-09 | 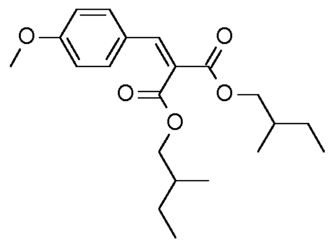 | | |

Manufacturing instruction:

Parts A and B are prepared separately and heated to 75°C. Under increasing stirring part B is incorporated into Part A and homogenized with Ultra Turrax for 10 seconds at 1000 rpm. The mixture is cooled down to room temperature under stirring and part C is added. At room temperature pH is adjusted between 5.5 and 6.0 with Part E.

Determination of Photostability

Four Quarz cuvettes (106-QS von Hellma 0,3mm inside surface sandblasted, outside surface polished) are filed with 0,0056g (± 0,0001g) of the cosmetic formulation to be analyzed. Then the samples are dryed for 15 min.

- Immediate value: After drying, each samples is rinsed with an appropriate solvent in 5 ml volumetric flask.
- Value after irradiation: After drying, samples are irradiated (Atlas CPE+ at 35°C) as following:

1h (2700 kJ/$m^2$)
2h (5400 kJ/$m^2$)
4h (10800 kJ/$m^2$)
10h (27000 kJ/$m^2$).

- After each irradiation time, each samples is rinsed with an appropriate solvent in 5 ml volumetric flask.

The samples are then analyzed with HPLC in order to measure the concentrations of the UV absorbers (Agilent 1100 Series).

Fig.1 shows the photostability of EHMC and MBM-09 respectively

Additional examples

| Ex-03: Sun Care Fluid W/O | | | | |
|---|---|---|---|---|
| Phase | Ingredients | INCI | % w/w | |
| | | | A* | B |
| A | Dehymuls® LE | PEG-30 Dipolyhydroxystearate | 3.50 | 3.50 |
| | Abil EM 90 | Cetyl PEG/PPG-10/1 Dimethicone | 1.00 | 1.00 |
| | DUB DIBA | Diisobutyl Adipate | 5.00 | 5.00 |
| | Cetiol® AB | C12-15 Alkyl Benzoate | 5.00 | 5.00 |
| | Arlamol HD | Isohexadecane | 3.00 | 3.00 |
| | Magnesium Stearate | Magnesium Stearate | 1.50 | 1.50 |
| | Uvinul® MC 80 | Ethylhexyl Methoxycinnamate | 10.00 | |
| | benzylidene malonate MBM-09 | | | 10.00 |
| | Uvinul® A Plus | Diethylamino Hydroxybenzoyl Hexyl Benzoate | 10.00 | 10.00 |
| | Uvinul® T 150 | Ethylhexyl Triazone | 2.50 | 2.50 |
| | Tinosorb® S | Bis-Ethylhexyloxyphenol Methoxyphenyl Triazine | 1.00 | 1.00 |
| | benzotriazole derivative BTD-01 | | | 1.20 |
| B | Water, demin. | Aqua | 47.30 | 47.30 |
| | Glycerin | Glycerin | 3.00 | 3.00 |
| | Magnesium Sulfate Heptahydrat e | Magnesium Sulfate | 1.00 | 1.00 |
| C | Xiameter PMX-0345 Cyclosiloxane Blend | Cyclopentasiloxane, Cyclohex asiloxane | 5.00 | 5.00 |
| | Perfume | Parfum | qs | qs |
| | Preservative | | qs | qs |
| *SPF* | | | 48.1 | 48.7 |
| UVA-PF | | | 23.4 | 23.3 |
| Colipa-ratio | | | 0.77 | 0.79 |
| Color of the formulation | | | yellowish | white |
| Manufacturing Instruction: Heat up phase A and B to 80°C. Add phase B slowly into phase A under increased stirring speed. Homogenize for a short time. Cool down under gentle stirring. Below 40°C add phase C. Finally homogenize for a short time again. *not according to the invention | | | | |

| Ex-04: Clear UV Protect Gel | | | | |
|---|---|---|---|---|
| Phase | Ingredients | INCI | % w/w | |
| | | | A* | B* |
| A | Cetiol® B | Dibutyl Adipate | 10.00 | 10.00 |
| | Finsolv EB | Ethylhexyl Benzoate | 15.00 | 15.00 |
| | Uvinul® MC 80 | Ethylhexyl Methoxycinnamate | 10.00 | |
| | | benzylidene malonate MBM-09 | | 10.00 |
| | Uvinul® A Plus | Diethylamino Hydroxybenzoyl Hexyl Benzoate | 10.00 | 10.00 |
| | Uvinul® T 150 | Ethylhexyl Triazone | 3.00 | 3.00 |
| | Tinosorb® S | Bis-Ethylhexyloxyphenol Methoxyphenyl Triazine | 2.00 | |
| | benzotriazole derivative BTD-01 | | | 2.00 |
| | Tinogard® TT | Tetradibutyl Pentaerithrityl Hyd roxyhydrocinnamate | 0.03 | 0.03 |
| B | Xiameter PMX-0245 Cyclopentasiloxane | Cyclopentasiloxane | 2.00 | 2.00 |
| C | Vitamin E-Acetate Care | Tocopheryl Acetate | 0.20 | 0.20 |
| D | Klucel M CS | Hydroxypropylcellulose | 2.50 | 2.50 |
| | Dermacryl-79 | Acrylates/Octylacrylamide Cop olymer | 0.50 | 0.50 |
| | Ethanol absolut pH.Eur. | Alcohol | 44.77 | 44.77 |
| SPF | | | 49.5 | 49.3 |
| UVA-PF | | | 23.2 | 22.5 |
| Colipa-ratio | | | 0.76 | 0.78 |
| Color of the formulation | | | yellowish | white |
| Manufacturing Instruction:<br>Heat phase A to 80°C, let stir for a while. Cool down to room temperature. Add phase B then C to phase A. Mix phase D. Incorporate phase A/B/C into D (oil phase into alcohol phase).<br>*not according to the invention | | | | |

| Ex-05: Easy Sun Spray | | | | |
|---|---|---|---|---|
| Phase | Ingredients | INCI | % w/w | |
| | | | A* | B |
| A | Eumulgin® VL 75 | Lauryl Glucoside, Polyglyceryl-2 Dipolyhydroxystearate, Glycerin | 3.00 | 3.00 |
| | Amphisol A | Cetyl Phosphate | 2.00 | 2.00 |
| | Cetiol® AB | C12-15 Alkyl Benzoate | 5.00 | 5.00 |
| | Uvinul® MC 80 | Ethylhexyl Methoxycinnamate | 10.00 | |
| | benzylidene malonate MBM-09 | | | 10.00 |
| | Uvinul® A Plus | Diethylamino Hydroxybenzoyl Hexyl Benzoate | 5.00 | 5.00 |
| | Tinosorb® S | Bis-Ethylhexyloxyphenol Methoxyphenyl Triazine | 2.50 | |
| | benzotriazole derivative BTD-01 | | | 2.50 |
| B | Water, demin. | Aqua | 61.10 | 61.10 |
| | Glycerin | Glycerin | 5.00 | 5.00 |
| | Avicel PC 611 | Microcrystalline Cellulose | 1.20 | 1.20 |
| | Edeta® BD | Disodium EDTA | 0.20 | 0.20 |
| | micronized (2-{4-[2-(4-Diethylamino-2-hydroxy-benzoyl)-benzoyl]-piperazine-1-carbonyl}-phenyl)-(4-diethylamino-2-hydroxy-phenyl)-methanone | | 4.00 | 4.00 |
| C | Triethanolamine Care | Triethanolamine | qs | qs |
| D | Xiameter PMX-0245 Cyclopentasiloxane | Cyclopentasiloxane | 5.00 | 5.00 |
| | Preservative | | qs | qs |
| | Perfume | Parfum | qs | qs |
| SPF | | | 35.7 | 33.3 |
| UVA-PF | | | 17.3 | 16.1 |
| Colipa-ratio | | | 0.80 | 0.83 |
| Color of the formulation | | | yellowish | white |
| Manufacturing Instruction:<br>Heat up the water of phase B to 80°C. Then add under high speed homogenization (Ultra Turrax type device) Edeta BD, Glycerin and Avicel PC611. Homogenize for 10 min at high speed. Add phase A into phase B at 80°C and homogenize. Neutralize with phase C to pH 5,5 - 6,0. Finally add phase D and homogenize shortly. Cool down to room temperature.<br>*not according to the invention | | | | |

| Ex-06: Very High Protection Sun Care Cream | | | | |
|---|---|---|---|---|
| Phase | Ingredients | INCI | % w/w | |
| | | | A* | B |
| A | Emulgade® PL 68/50 | Cetearyl Glucoside, Cetearyl Alcohol | 4.00 | 4.00 |
| | Eumulgin® SG | Sodium Stearoyl Glutamate | 1.00 | 1.00 |
| | Dehymuls® PGPH | Polyglyceryl-2 Dipolyhydroxystearate | 1.00 | 1.00 |
| | Cetiol® B | Dibutyl Adipate | 10.00 | 10.00 |
| | Cetiol® AB | C12-15 Alkyl Benzoate | 2.00 | 2.00 |
| | Cetiol® CC | Dicaprylyl Carbonate | 2.00 | 2.00 |
| | Uvinul® MC 80 | Ethylhexyl Methoxycinnamate | 10.00 | |
| | benzylidene malonate MBM-09 | | | 10.00 |
| | Uvinul® A Plus | Diethylamino Hydroxybenzoyl Hexyl Benzoate | 8.50 | 8.50 |
| | Tinosorb® S | Bis-Ethylhexyloxyphenol Methoxyphenyl Triazine | 3.00 | |
| | benzotriazole derivative BTD-01 | | | 3.00 |
| B | Water, demin. | Aqua | 24.90 | 24.90 |
| | Glycerin | Glycerin | 3.00 | 3.00 |
| | Rheocare® XG | Xanthan Gum | 0.40 | 0.40 |
| | Edeta® BD | Disodium EDTA | 0.20 | 0.20 |
| C | Tinosorb® A2B | Tris-Biphenyl Triazine (nano), Aqua, Decyl Glucoside, Butylene Glycol, Disodium Phosphate, Xanthan Gum | 13.00 | 13.00 |
| | Tinosorb® M | Methylene Bis-Benzotriazolyl Tetramethylbutylphenol (nano), Aqua, Decyl Glucoside, Propylene Glycol, Xanthan Gum | 10.00 | 10.00 |
| D | Xiameter PMX-0345 Cyclosiloxane Blend | Cyclopentasiloxane, Cyclohexasiloxane | 5.00 | 5.00 |
| | Tospearl A145 | Polymethylsilsesquioxane | 2.00 | 2.00 |
| | Preservative | | qs | qs |
| | Perfume | Parfum | qs | qs |
| SPF | | | *80.2* | *106.2* |
| UVA-PF | | | *37.4* | *36.2* |
| Colipa-ratio | | | *0.84* | *0.84* |
| Manufacturing Instruction: Heat up phase A to and phase B to 80°C. Incorporate phase A into phase B under stirring, homogenize. Add phase C under stirring, homogenize. Cool down under stirring to RT. Add the phase D under stirring, homogenize. *not according to the invention | | | | |

<table>
<tr><td colspan="5">Ex-07: Face Cream Ageless</td></tr>
<tr><td rowspan="2">Phase</td><td rowspan="2">Ingredients</td><td rowspan="2">INCI</td><td colspan="2">% w/w</td></tr>
<tr><td>A*</td><td>B</td></tr>
<tr><td rowspan="15">I</td><td>Eumulgin® Prisma</td><td>Disodium Cetearyl Sulfosuccinate</td><td>0.50</td><td>0.50</td></tr>
<tr><td>Cutina® PES</td><td>Pentaerythrityl Distearate</td><td>1.00</td><td>1.00</td></tr>
<tr><td>Cutina® HVG</td><td>Hydrogenated Vegetable Glycerides</td><td>1.50</td><td>1.50</td></tr>
<tr><td>Lanette® O</td><td>Cetearyl Alcohol</td><td>1.00</td><td>1.00</td></tr>
<tr><td>Cetiol® B</td><td>Dibutyl Adipate</td><td>4.00</td><td>4.00</td></tr>
<tr><td>Myritol® 331</td><td>Cocoglycerides</td><td>4.00</td><td>4.00</td></tr>
<tr><td>Cetiol® Sensoft</td><td>Propylheptyl Caprylate</td><td>3.00</td><td>3.00</td></tr>
<tr><td>Copherol® F 1300 C</td><td>Tocopherol</td><td>0.50</td><td>0.50</td></tr>
<tr><td>Cosmedia® SP</td><td>Sodium Polyacrylate</td><td>0.80</td><td>0.80</td></tr>
<tr><td>Rheocare® XG (Europe only)</td><td>Xanthan Gum</td><td>0.10</td><td>0.10</td></tr>
<tr><td>Parsol 1789</td><td>Butyl Methoxydibenzoylmethane</td><td>3.00</td><td>3.00</td></tr>
<tr><td>Tinosorb® S</td><td>Bis-Ethylhexyloxyphenol Methoxyphenyl Triazine</td><td>1.00</td><td></td></tr>
<tr><td>benzotriazole derivative BTD-01</td><td></td><td></td><td>1.00</td></tr>
<tr><td>Uvinul® N 539T</td><td>Octocrylene</td><td>2.00</td><td></td></tr>
<tr><td>benzylidene malonate MBM-09</td><td></td><td></td><td>2.00</td></tr>
<tr><td rowspan="4">II</td><td>Glycerin</td><td>Glycerin</td><td>5.00</td><td></td></tr>
<tr><td>Tinosorb® M</td><td>Methylene Bis-Benzotriazolyl Tetramethylbutylphenol (nano), Aqua, Decyl Glucoside, Propylene Glycol, Xanthan Gum</td><td>6.00</td><td></td></tr>
<tr><td></td><td>micronized 5,6,5',6'-tetraphenyl-3,3'-(1,4-Phenylene) bis-(1,2,4-Triazine)</td><td></td><td>6.00</td></tr>
<tr><td>Water, demin.</td><td>Aqua</td><td>66.60</td><td>66.60</td></tr>
<tr><td rowspan="2">III</td><td>Preservative</td><td></td><td>qs</td><td>qs</td></tr>
<tr><td>Perfume</td><td>Parfum</td><td>qs</td><td>qs</td></tr>
<tr><td colspan="3">SPF</td><td>22.3</td><td>24.6</td></tr>
<tr><td colspan="3">UVA-PF</td><td>14.9</td><td>14.6</td></tr>
<tr><td colspan="3">Colipa-ratio</td><td>0.97</td><td>0.95</td></tr>
<tr><td colspan="5">Manufacturing Instruction:<br>Heat phase I to 80-85°C and stir until uniform. Heat phase II separately to 80-85°C and add to phase I while stirring. Allow the emulsion to cool while stirring in such a way that it remains in continuous motion. Avoid incorporation of air. Homogenize with a suitable dispersion unit (e.g. Ultra Turrax) at approx.55°C. Stir while cooling to 40°C and add phase III. Stop stirring at 30°C.<br>*not according to the invention</td></tr>
</table>

Method to assess in vitro Sun Protection Factor measurement (SPF)

End-product application rate 1.3 mg/cm$^2$ on sandblasted PMMA plates (Helioscience ®) UV Transmittance analysis with Labsphere UV-1000S Transmittance Analyser

$$SPF = \frac{\int_{290nm}^{400nm} E_\lambda \cdot S_\lambda \cdot d\lambda}{\int_{290nm}^{400nm} E_\lambda \cdot S_\lambda \cdot T_\lambda \cdot d\lambda}$$

wherein $E_\lambda$ = erythema action spectrum;$S_\lambda$ = solar spectral irradiance and $T_\lambda$ = spectral transmittance of the sample.

Method to assess in vitro UVA protection factor (UVA PF)

End-product application rate 1.3 mg/cm$^2$ on sandblasted PMMA plates (Helioscience ®) UV Transmittance analysis with Labsphere UV-1000S Transmittance Analyser Pre-irradiation step (to take the sun care product photostability into account) via a solar simulator such as Atlas Suntest CPS+

$$\mathrm{PF\,UVA} = \frac{\sum_{320}^{400} \Delta\lambda}{\sum_{320}^{400} T_\lambda \cdot \Delta\lambda} = \frac{1}{T_m}$$

wherein $T_\lambda$ = sunscreen product transmittance at wavelength $\lambda$ and $T_m$ = mean arithmetical value of Transmittance data in the UVA range.

## Claims

**1.** Use of the benzotriazole derivative (a) corresponding to the formula

(1) 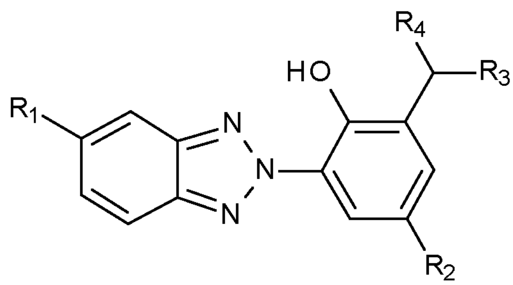

wherein

$R_1$ is hydrogen; $C_1$-$C_5$alkyl; $C_1$-$C_5$alkoxy; or halogen;
$R_2$ is hydrogen; $C_1$-$C_{20}$alkyl; $C_1$-$C_5$alkoxy; $C_1$-$C_5$alkoxycarbonyl; $C_5$-$C_{10}$cycloalkyl; $C_5$-$C_{10}$aryl; or $C_5$-$C_{10}$aralkyl;
$R_3$ is $C_1$-$C_{20}$alkyl; $C_5$-$C_{10}$cycloalkyl; $C_1$-$C_{20}$alkoxy; or $C_5$-$C_{10}$cycloalkoxy; and
$R_4$ is hydrogen; or $C_1$-$C_5$alkyl;
for photo-stabilizing cosmetic formulations comprising at least one of the UV filters ($c_6$) Ethylhexyl Methoxycinnamate; and
($b_1$) a compound of formula

MBM-09

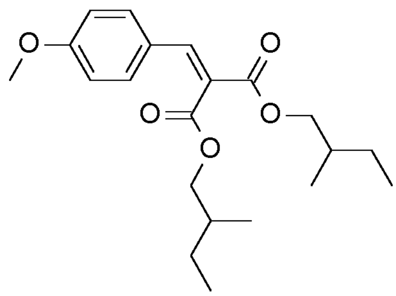

2. Use according to claim 1 wherein the benzotriazole derivative (a) is 2-(2H-Benzotriazol-2-yl)-6-(2-ethylhexyloxymethyl)-4-methyl-phenol.

**Patentansprüche**

1. Verwendung des Benzotriazolderivats (a) gemäß der Formel

(1)

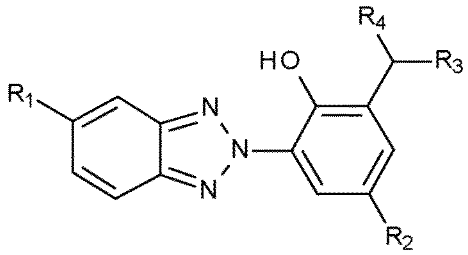

wobei

$R_1$ für Wasserstoff; $C_1$-$C_5$-Alkyl; $C_1$-$C_5$-Alkoxy oder Halogen steht;
$R_2$ für Wasserstoff; $C_1$-$C_{20}$-Alkyl; $C_1$-$C_5$-Alkoxy; $C_1$-$C_5$-Alkoxycarbonyl; $C_5$-$C_{10}$-Cycloalkyl; $C_5$-$C_{10}$-Aryl oder $C_5$-$C_{10}$-Aralkyl steht;
$R_3$ für $C_1$-$C_{20}$-Alkyl; $C_5$-$C_{10}$-Cycloalkyl; $C_1$-$C_{20}$-Alkoxy oder $C_5$-$C_{10}$-Cycloalkoxy steht und
$R_4$ für Wasserstoff oder $C_1$-$C_5$-Alkyl steht;
zur Photostabilisierung von kosmetischen Formulierungen, die mindestens einen der UV-Filter
($c_6$) Ethylhexyl-Methoxycinnamat und
($b_1$) eine Verbindung der Formel

MBM-09

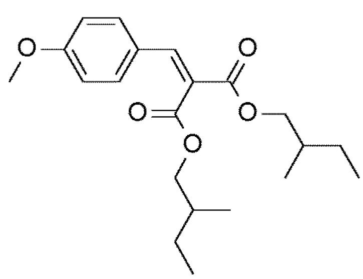

umfassen.

2. Verwendung nach Anspruch 1, wobei es sich bei dem Benzotriazolderivat (a) um 2-(2H-Benzotriazol-2-yl)-6-(2-ethylhexyloxymethyl)-4-methylphenol handelt.

## Revendications

1. Utilisation du dérivé de benzotriazole (a) correspondant à la formule

(1) 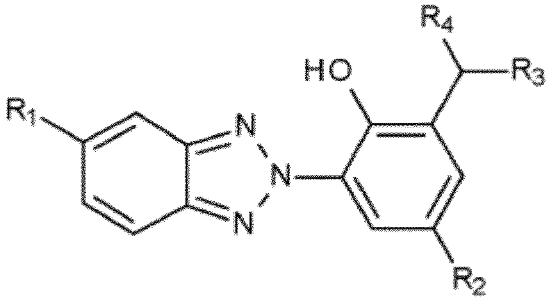

$R_1$ étant hydrogène ; $C_1$-$C_5$alkyle ; $C_1$-$C_5$alcoxy ; ou halogène ;
$R_2$ étant hydrogène ; $C_1$-$C_{20}$alkyle ; $C_1$-$C_5$alcoxy ; $C_1$-$C_5$alcoxycarbonyle ; $C_5$-$C_{10}$cycloalkyle ; $C_5$-$C_{10}$aryle ; ou $C_5$-$C_{10}$aralkyle ;
$R_3$ étant $C_1$-$C_{20}$alkyle ; $C_5$-$C_{10}$cycloalkyle ; $C_1$-$C_{20}$alcoxy ; ou $C_5$-$C_{10}$cycloalcoxy ; et
$R_4$ étant hydrogène ; ou $C_1$-$C_5$alkyle ;
pour la photostabilisation de formulations cosmétiques comprenant au moins l'un des filtres UV ($c_6$) méthoxy-cinnamate d'éthylhexyle ; et
($b_1$) un composé de formule
0000076117EP02 / RecID 81 - 2 -

MBM-09 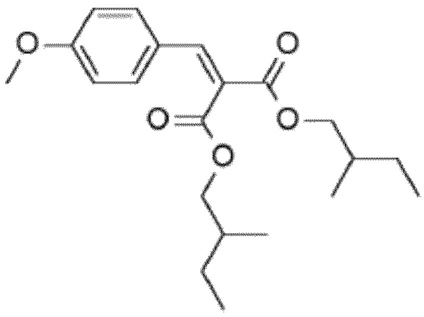

.

2. Utilisation selon la revendication 1, le dérivé de benzotriazole (a) étant le 2-(2H-benzotriazol-2-yl)-6-(2-éthylhexyloxyméthyl)-4-méthyl-phénol.

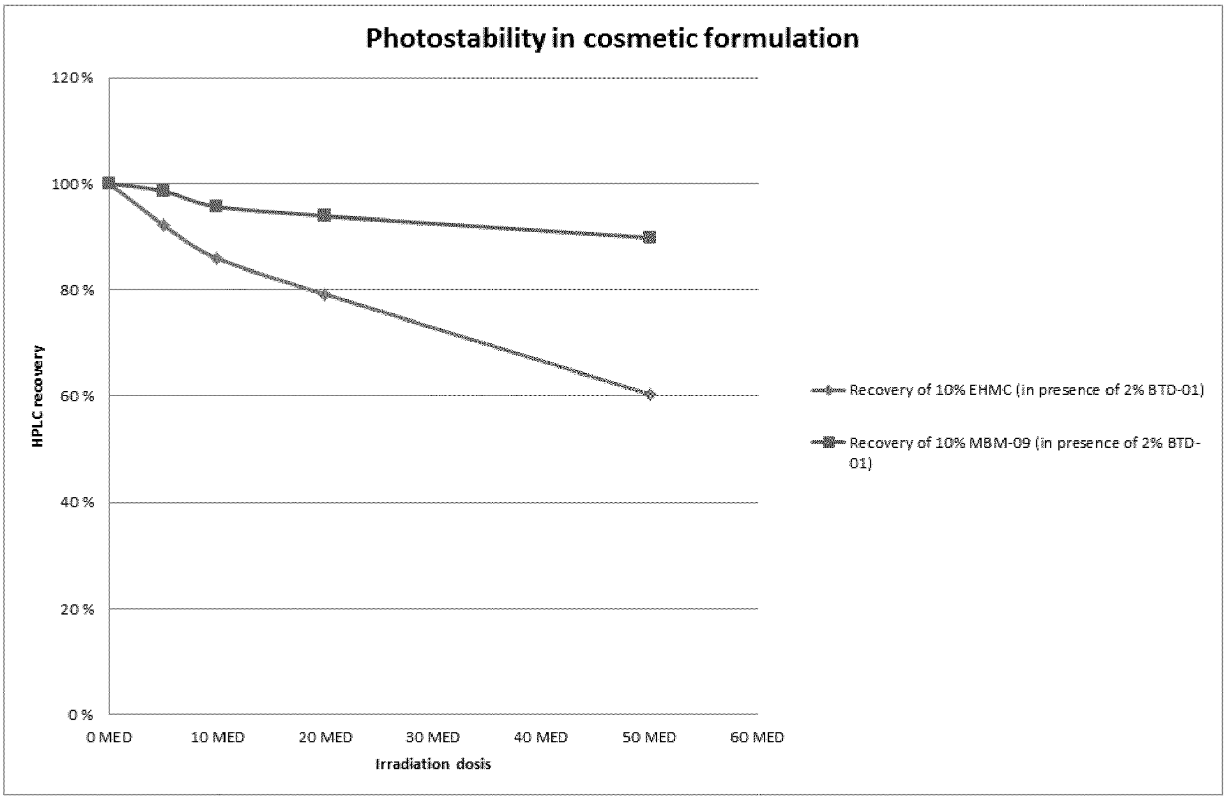


Fig. 1: Photostability of EHMC and MBM-09 respectively

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060052491 A
- EP 2078521 A1
- US 4457911 A
- WO 0341675 A
- WO 2002039974 A
- DE 10229995
- EP 1371358 A
- EP 1371357 A
- EP 1371356 A
- DE 10138496
- US 20040247536 A
- DE 102007035567
- WO 2009012871 A
- DE 1165574 A
- DE 2024051 A
- FR 2252840 A


### Non-patent literature cited in the description


- **TODD et al.** of suitable volatile silicones may in addition be found in. *Cosm. Toil.,* 1976, vol. 91 (27
- *J. Soc. Cosm. Chem.,* 1973, vol. 24, 281
- *J. Pharm. Pharmacol.,* 1975, vol. 26, 531
- **R. LOCHHEAD.** *Cosm. Toil.,* 1993, vol. 108, 95